# EUROPEAN PATENT APPLICATION

(11) **EP 3 892 329 A1**
(43) Date of publication of application: **13.10.2021**
(21) Application number: 20315119.6
(22) Date of filing: 06.04.2020
(51) Int. Cl.: A61P 9/10, A61K 36/02

(54) **COMPOSITION FOR USE IN PREVENTING ATHEROSCLEROSIS DEVELOPPMENT**

(71) Applicant: Algosource, 44602 Saint-Nazaire Cedex (FR)
(72) Inventor: Lepine, Olivier, 44600 Saint-Nazaire (FR); Ouguerram, Khadija, 44100 Nantes (FR)
(74) Representative: Ipsilon

(57) **Abstract**

Composition for use in protecting mammalian subjects against atherosclerosis development, in particular against atheroma formation or in atheroma reduction, by oral administration of an aqueous liquid extract of spirulina including phycocyanin. Said composition can be used in protecting genetically hypercholestolemic mammalian subjects against atherosclerosis development. Moreover, said composition can be used especially for protecting offspring mammalian subjects against atherosclerosis development, by oral administration to mothers of the offspring subjects of said composition at least during a part of the gestation period of the offspring mother or at least during part of the gestation period and of the lactation period.

SEQUENCE LISTING

<110>  ALGOSOURCE

<120>  Composition for use in preventing atherosclerosis development

<130>  PN001051

<160>  4

<170>  PatentIn version 3.5

<210>  1
<211>  22
<212>  DNA
<213>  artificial sequence

<220>
<223>  Peroxisome proliferator-activated receptor alpha - F primer

<400>  1
cgtttgtggc tggtcaagtt cg                                                22

<210>  2
<211>  22
<212>  DNA
<213>  artificial sequence

<220>
<223>  Peroxisome proliferator-activated receptor alpha - r primer

<400>  2
agtggggaga gaggacagat gg                                                22

<210>  3
<211>  22
<212>  DNA
<213>  artificial sequence

<220>
<223>  Tata box binding protein - F primer

<400>  3
acttcgtgca agaaatgctg aa                                                22

<210>  4
<211>  20
<212>  DNA
<213>  artificial sequence

<220>
<223>  Tata box binding protein - R primer

<400>  4
gcagttgtcc gtggctctct                                                   20

## Description

### FIELD OF THE INVENTION

The present invention belongs to the field of atherosclerosis prevention. In particular the present invention relates to a composition for use in protecting mammalian subjects against atherosclerosis development.

### BACKGROUND OF THE INVENTION

Atherosclerosis is a disease coming from accumulation, in the inner walls of arteries, of lipid debris and fibrous tissue leading to the formation of an atheromatous plaque, also named atheroma, which narrows the arteries and restricts blood flow. Atherosclerosis is a major cause of disability and vascular death worldwide. This multifactorial disease is the consequence of the interplay of genetic and environmental factors.

Several therapeutic strategies targeting risk factors can be used to limit aggravation in the process of atherosclerosis but the prevention remains the most effective strategy to counter the development of this disease. For instance, changes in diet and exercise can help prevent the development of atherosclerosis. Some of the known treatments include for instance medications to lower cholesterol in blood, such as statins, or to reduce blood pressure, or to decrease clotting, such as aspirin.

However, some misunderstanding persists, for example, in case of severe familial forms of hypercholesterolemia, some patients are paradoxically protected from vascular complications without clear genetic, biological or environmental explanations.

### AIMS OF THE INVENTION

A first aim of the invention is to provide a composition which can be used in protecting mammalian subjects against atherosclerosis development.

A second aim of the invention is to provide a composition which can also be used in protecting genetically hypercholestolemic mammalian subjects against atherosclerosis development.

Another aim of the invention is to provide a composition which can also be used in protecting progeny of mammalian subjects against atherosclerosis development.

Still another aim of the invention is to provide a composition that can be administrated easily, such as orally, to the subjects in need thereof.

### DESCRIPTION OF THE INVENTION

The inventors have found a composition which is surprisingly effective to prevent atherosclerosis development in mammalian subjects, and is particularly effective against atheroma formation and atheroma reduction.

Consequently, the present invention relates to a composition for use in protecting mammalian subjects against atherosclerosis development, in particular against atheroma formation or in atheroma reduction, by oral administration of said composition comprising an aqueous liquid extract including phycocyanin.

According to a particular embodiment of the invention the composition can be used for protecting genetically hypercholestolemic mammalian subjects against atherosclerosis development.

In addition, during their research work to understand the impact of maternal health on the occurrence of clinical diseases in offsprings, the inventors have found that the same composition is surprinsingly also effective to prevent atherosclerosis development in offsprings of mammalian subjects.

Therefore, the present invention also relates to a composition for use in protecting offspring mammalian subjects against atherosclerosis development, by oral administration to mothers of the offspring subjects of said composition comprising an aqueous liquid extract including phycocyanin.

Said composition can be administrated at least during a part of the gestation period of the offspring mother, or can be administrated at least during part of the gestation period and of the lactation period of the offspring mother.

According to a preferred embodiment of the present invention the aqueous liquid extract of the composition comprises from 0.2 mg to 15 mg, preferably from 0.5 to 12 mg, more preferably from 0.8 to 11 mg of phycocyanin, per mL of said extract.

A preferred aqueous liquid extract containing phycocyanin is prepared by water extraction from spirulina according to the process of FR 3 064 269 wherein said extract contains non denaturated phycocyanin, that is phycocyanin which, in particular, has not being subjected to a drying step. The preferred source of phycocyanin is spirulina, for instance spirulina *Arthrospira platensis, Aphanizomenon flos-aquae, or Phormidium molle,* but any other source of phycocyanin could be used.

Said composition of the present invention can also comprise polysaccharides from spirulina.

According to a preferred embodiment of the present invention the aqueous liquid extract of the composition comprises from 0.5 mg to 0.25 mg, preferably from 0.10 to 0.20 mg of polysaccharides per mL of the aqueous liquid extract, preferably polysaccharides from spirulina.

According to a particular embodiment the composition of the present invention is in liquid form and consists of essentially said aqueous liquid extract.

Said composition could, for example, be added in beverage water, or in food, like a dietary supplement.

The composition of the invention can be administrated to mammalian animal or to human subjects. Said composition can be administered at a dosage regime of between 1 mg and 100 mg phycocyanin /weight kg/day , preferably between 5 mg and 80 mg phycocyanin /weight kg/day, of the mammalian subject or the offspring mother.

The preferred dosage regime for human beings is between 1 to 15 mg phycocyanin /weight kg/day, more preferably between 5 to 10 mg phycocyanin /weight kg/day.

### FIGURES

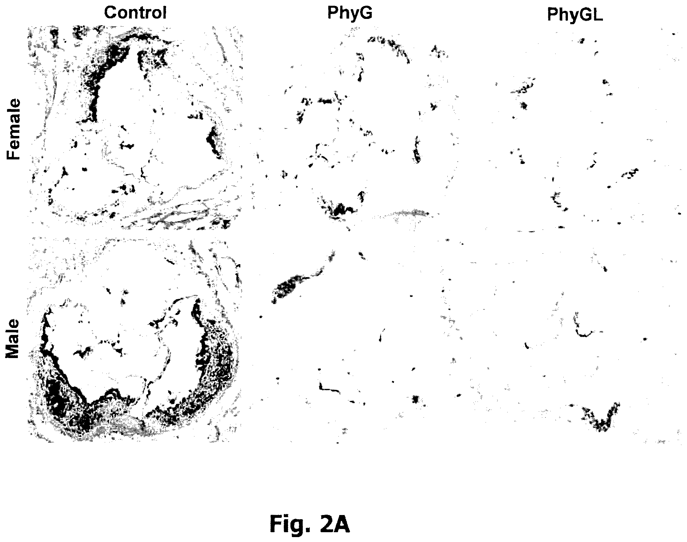

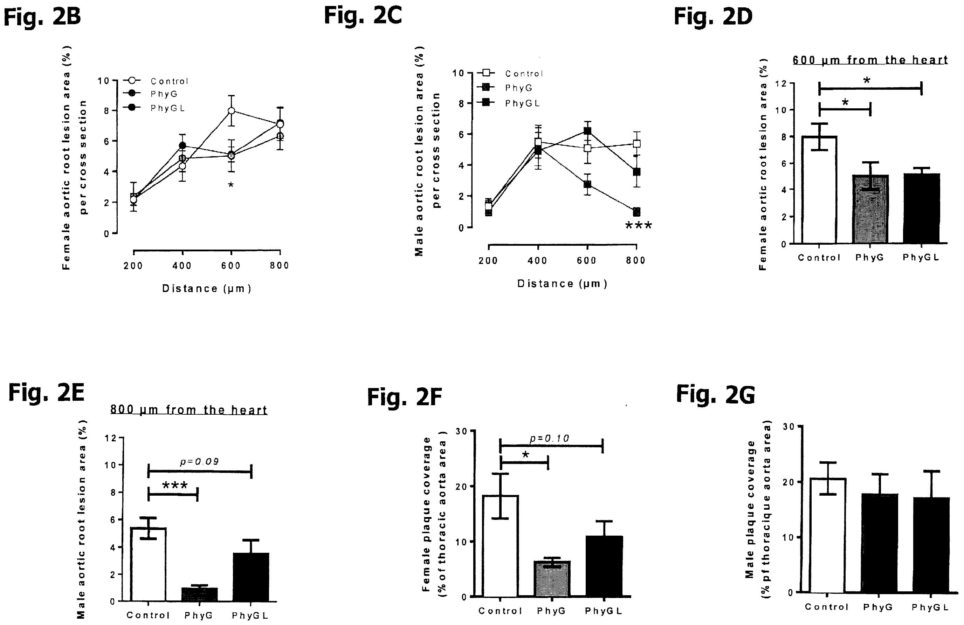

The invention will be further described in the below embodiments given with reference to the accompanying drawings, in which:

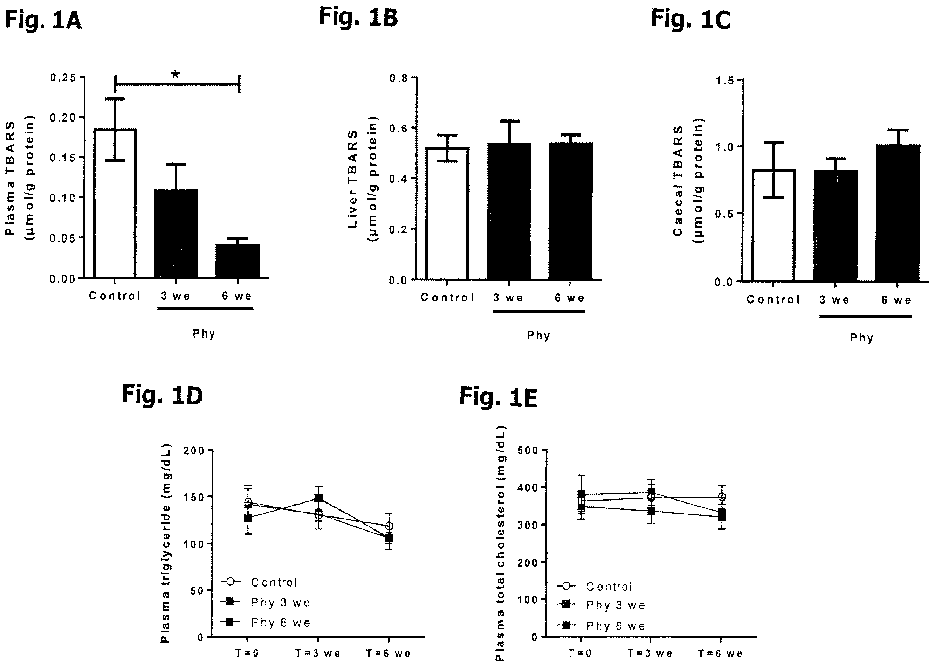

Figures 1A, 1B, 1C, 1D and 1E illustrate the effect of direct C-Phycocyanin administration on oxidative stress and plasma lipids in female ApoE KO mice : Thiobarbituric acid reactive substances (TBARS) measured in plasma (Fig. 1A), in liver (Fig.1B) and in caecal supernatant (Fig.1C) of female adult ApoE KO mice receiving non-supplemented water (Control) or C-Phycocyanin concentrate in drinking water (Phy) during 3 or 6 weeks (n=5-6). Follow up of plasma triglyceride (Fig. 1D) and total cholesterol (Fig.1E) in those same mice.
Figures-2A to 2G illustrate the effect of perinatal C-Phycocyanin supplementation on offspring atherosclerosis development in adult life: Representative oil red O/hematoxylin staining in aortic root cross-sections from adult ApoE KO born to control or supplemented mothers (Fig. 2A). Quantification of the aortic root oil red O/hematoxylin staining for female pups at all distances from the heart (Fig. 2B) and specifically at 600 µm (n=6-10) (Fig. 2D) and for male pups at all distances from the heart (Fig. 2C) and at 800 µm (n=7-10) (Fig. 2E). Quantification of the thoracic aorta oil red O/hematoxylin staining for female descendants (n=6-7) (Fig. 2F) and for male descendants (n=4-10) (Fig. 2G).

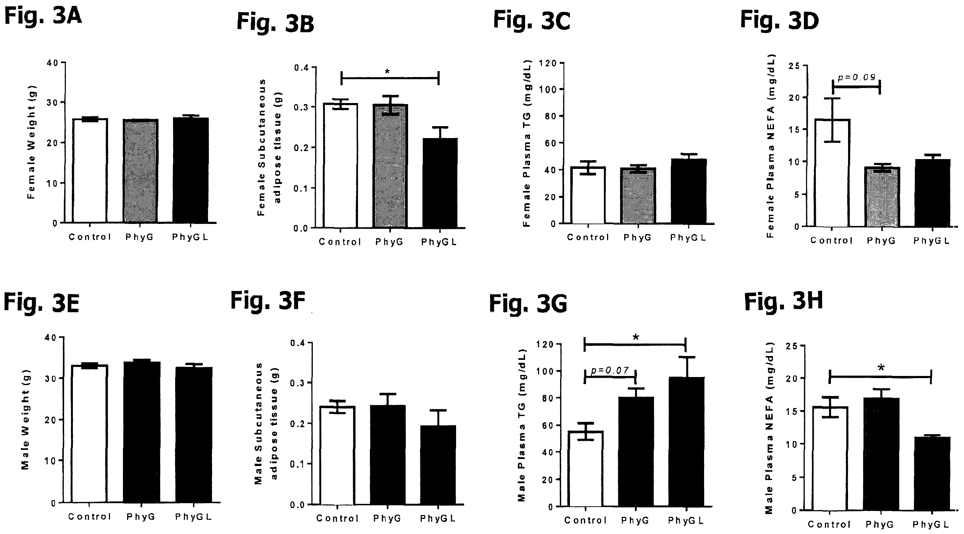

Figures 3A to 3H show the body composition and plasma lipid profiles in progeny of ApoE KO mice: Final body weight and subcutaneous adipose tissue at 25 weeks old in female (n=6-12) (Fig. 3A and Fig. 3B) and male (n=8-11) (Fig. 3E and Fig. 3F) pups. Plasma triglycerides (TG) and non-esterified fatty acids (NEFA) in female (n=6-12) (Fig. 3C and Fig. 3D) and male (n=8-11) (Fig. 3G and Fig. 3H) descendants.

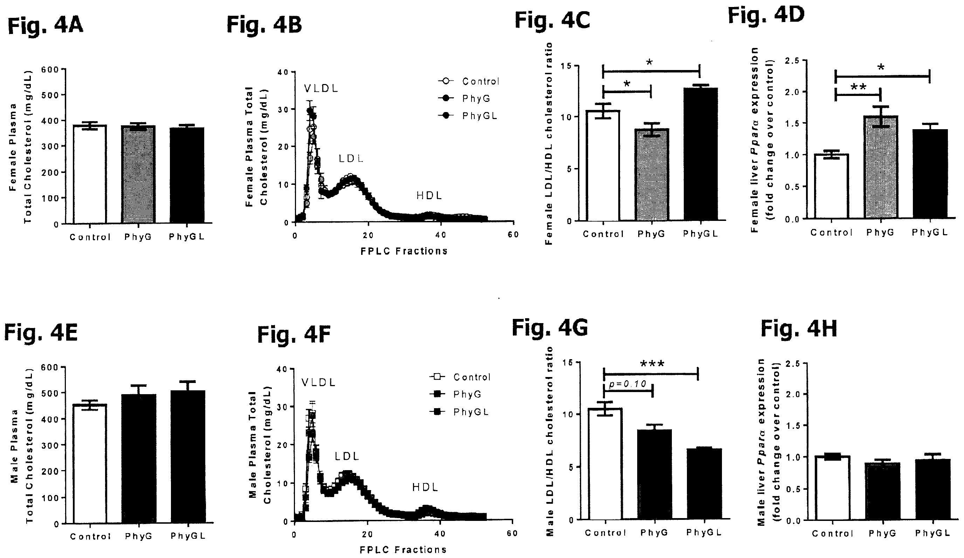

Figures 4A to 4H illustrate the impact of perinatal antioxidant administration on plasma cholesterol and lipoprotein fractions of progeny: Plasma total cholesterol in female (n=6-12) and male (n=8-11) pups respectively before (Fig. 4A and Fig. 4E) and after (n=3-4) (Fig. 4B and Fig. 4F) fast protein liquid chromatography (FPLC) enabling very low density lipoprotein (VLDL), low density lipoprotein (LDL) and high density lipoprotein (HDL) separation and the calculation of LDL-to-HDL ratio (Fig. 4C and Fig. 4G). Liver gene expression of *Peroxisome proliferator-activated receptor a (Ppar a*) in female (n=6-12) and male (n=8-11) (Fig. 4D and Fig. 4H) offspring.

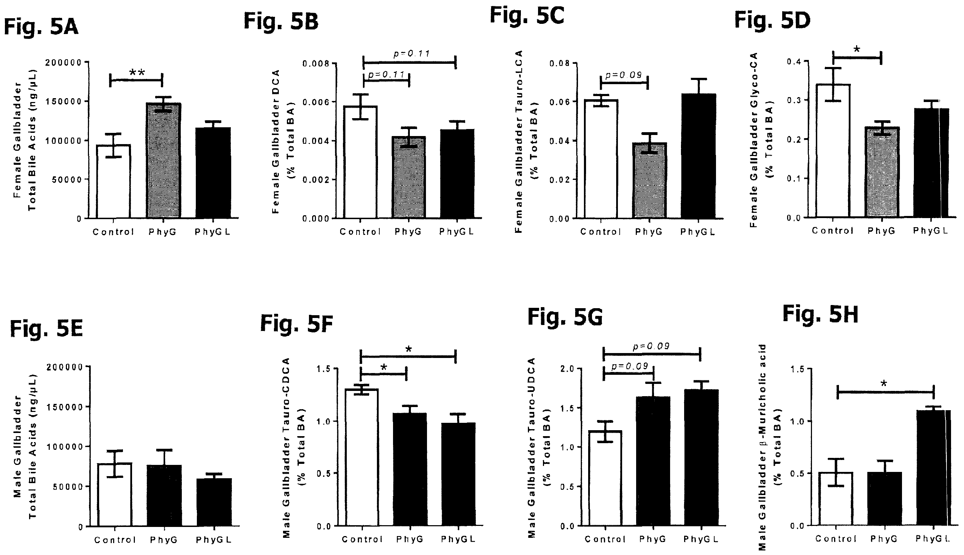

Figures 5A to 5H present the sexual dimorphism effect of C-Phycocyanin supplementation during gestation and/or lactation on progeny bile acid composition. Total bile acids (Fig. 5A), deoxycholic acid (DCA) (Fig. 5B), Tauro-lithocholic acid (LCA) (Fig. 5C) and Glyco-cholic acid (CA) (Fig. 5D) in gallbladder of female offspring (n=6-7). Total bile acids (Fig. 5E), Tauro-chenodeoxycholic acid (CDCA) (Fig. 5F), Tauro-ursodeoxycholic acid (UDCA) (Fig. 5G) and β-muricholic acid acid (B-MCA) (Fig. 5H) in gallbladder of male offspring (n=5-8).

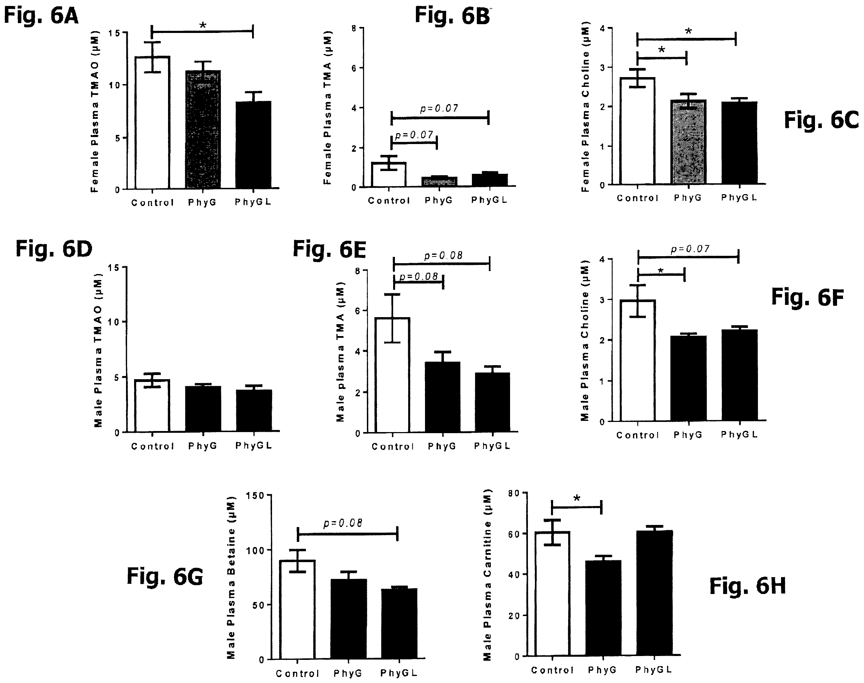

Figures 6A to 6H show the perinatal antioxidant administration effects on trimethylamine-N-oxide and its precursor levels. Female offspring trimethylamine-N-oxide (TMAO) (Fig. 6A), trimethylamine (TMA) (Fig. 6B) and choline (Fig. 6C) measured in plasma at 16 weeks old (n=6-12). Male offspring trimethylamine-N-oxide (TMAO) (Fig. 6D), trimethylamine (TMA) (Fig. 6E), choline (Fig. 6F), betaine (Fig. 6G) and carnitine (Fig. 6H) measured in plasma at 16 weeks old (n=8-11).

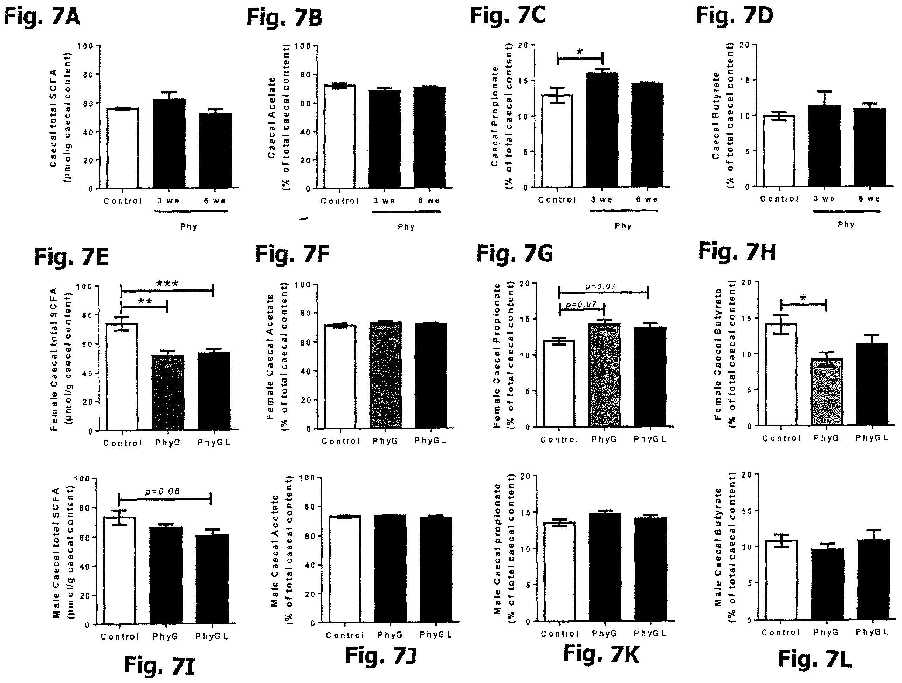

Figures 7A to 7L illustrate the effect of C-Phycocyanin administered during gestation and/or lactation on SCFA production in mothers and offspring. Total short chain fatty acids (SCFA) (Fig. 7A, Fig. 7E and Fig. 7I), acetate (Fig. 7B, Fig. 7F and Fig. 7J), propionate (Fig. 7C, Fig. 7G and Fig. 7K) and butyrate (Fig. 7D, Fig. 7H and Fig. 7L) content measured at 25 weeks old in caecal supernatant in mothers (n=5-6) and female (n=6-12) and male (n=8-11) offspring respectively. Female adult ApoE KO mice received non supplemented water (Control) or C-Phycocyanin concentrate in drinking water (Phy) during 3 or 6 weeks.
   In all the Figures, above, and the text of the application, PhyG indicates offspring born to mothers who have received C-phycocyanin concentrate during gestation; PhyGL, offspring born to mothers who have received C-phycocyanin concentrate during gestation and lactation.

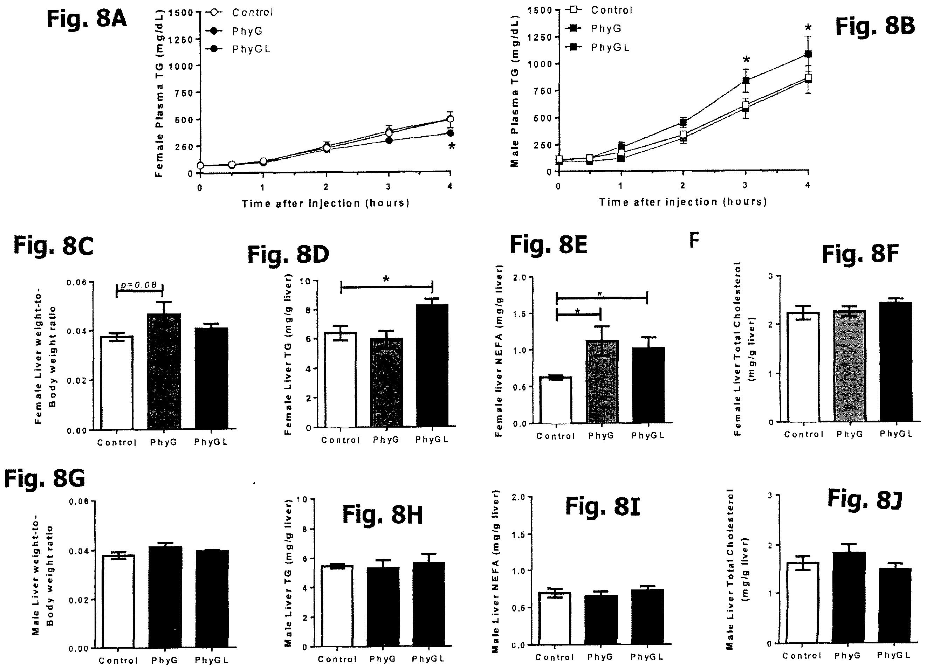

Figures 8A to 8J illustrate the perinatal antioxidant supplementation on hepatic lipid composition of progeny: Follow-up of hepatic triglyceride production rate after Triton injection measured at 20 weeks old in female (n=6-9) (Fig.8A) and male (n=5-9) (Fig.8B) pups of ApoE KO mice. Liver weight-to-body weight ratio (Fig.8C and Fig.8G), hepatic TG (Fig.8D and Fig.8H), NEFA (Fig.8E and Fig.8I) and total cholesterol (Fig.8F and Fig.8J) content in female (n=6) and male (n=6) offspring respectively.

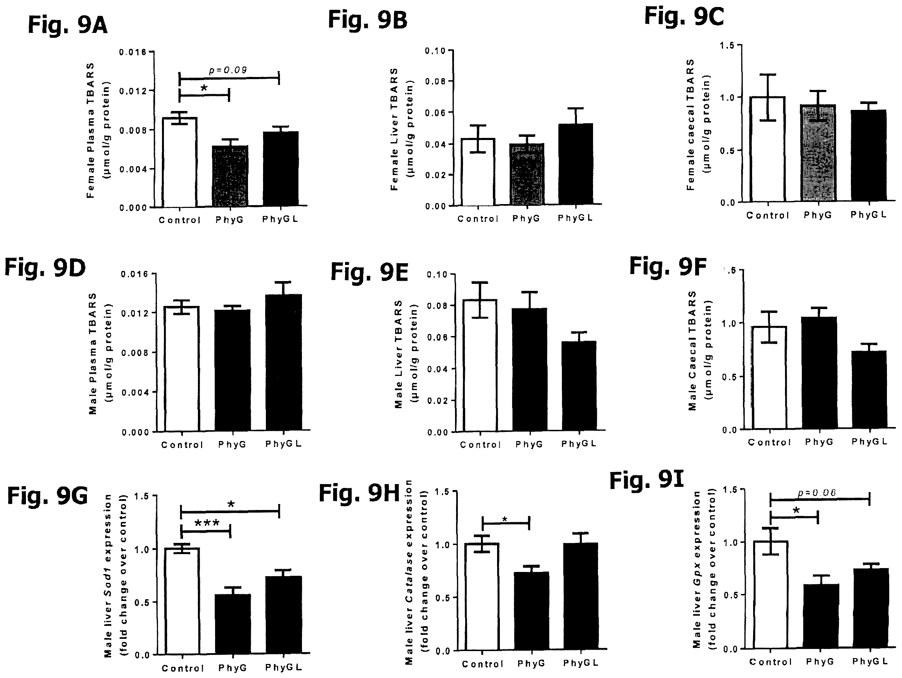

Figures 9A to 9I show the impact of C-Phycocyanin supplementation during gestation and/or lactation on lipid peroxidation marker of offspring: TBARS measured at 25 weeks old in plasma (Fig.9A and Fig.9D), in liver (Fig.9B and Fig.9E), and in caecal supernatant (Fig.9C and Fig.9F) in female (n=6-12) and male (n=8-11) offspring respectively. Hepatic gene expression of *Superoxide dismutase 1 (Sod1)* (Fig. 9G), *Catalase* (Fig.9H) and *Glutathione peroxidase (Gpx)* (Fig.9I) in male pups are also shown.

### EXEMPLES

### Materials and Methods

### Experiments were carried out on

### Concentrate of C-Phycocyanin

Experiments were all carried out with a concentrate of phycocyanin (Phy) which is a patented water extract of *Arthrospira platensis* (Number of patent : 17 52452; AlgoSource Technologies, Saint Nazaire, France) and corresponds to the alga cytoplasmic hydrophilic compounds, concentrated in Phy as presented in Table 1 below. This concentrate is an extract made in only water and has not incurred heating. Phycocyanin is therefore not denatured, nor degraded.

This concentrate contains also polysaccharides in the proportions given in table 1 below.

**Table 1**

| **Compounds in extract** | **Amount (mg for 100 mL of aqueous extract)** |
|---|---|
| Phycocyanin | 88.3 |
| Total carbohydrate | 400 |
| Total fat mass | undetectable |
| Amino acids | |
| threonine | 9 ± 1 |
| aspartate | 19 ± 3 |
| alanine | 17 ± 2 |
| arginine | 14 ± 2 |
| leucine | 16 ± 2 |
| glutamate | 22 ± 3 |
| Vitamin B12 | undetectable |
| Calcium | 33 ± 7 |
| Potassium | 6 ± 1 |
| Iron | 0.1 ± 0.04 |
| Copper | 0.02 ± 0.01 |
| Magnesium | 2 ± 0.4 |

| | |
|---|---|
| Energic value : 2 kcal/100 g | |

### Mice and Diets

For reproduction, one apolipoprotein E knock out C57Bl/6.129P2-APOE/J (ApoE KO) male mice was housed with 2 female ApoE KO to allow reproduction (Charles River Laboratories, France). Mice had *ad libitum* access to water and food with constant 12/12h light/dark cycles. Mice were fed with a chow diet (A04; Safe Diets, France). Pregnant ApoE KO mice received a concentrate of Phy in drinking water (80 mg Phy/kg body weight/day) during their 3 weeks of gestation (PhyG) or during their 6 weeks of gestation and lactation (PhyGL). At weaning, at 21 days old, female and male progeny were separated, fed with a chow diet and killed at 25 weeks of age. All experimental procedures were approved by the Committee of Ethics in Animal Experiments of Pays de la Loire, France (Project No. APAFIS#6819) and performed according to the European Union regulations for the care and use of animals for experimental procedures (2010/63/EU).

Several tests were performed:

### Hepatic triglyceride production

At 19 weeks old, offspring were injected intraperitoneally with Triton at 600 mg/kg body weight (Sigma-Aldrich, France) after 4h of fasting. Immediately prior to injection and at 0.5, 1, 2, 3 and 4 hours following injection, blood from tail vein were collected in EDTA K2 tubes (Sarsted, Germany) and plasma TG were quantified using enzymatic assay (Dyasis, France).

### Blood analyses and tissue collection

At 16 weeks, offspring's blood were collected from tail vein after 4h of fasting into tubes containing EDTA K2 (Sarsted, Germany) to determine level of trimethylamine-N-oxide (TMAO) and its precursors. Plasma choline, betaine, carnitine, TMA and TMAO were analyzed using Ultra Performance Liquid Chromatography (Waters, Manchester, UK) on a hydrophilic interaction liquid chromatography HILIC-BEH column coupled to a Waters Xevo TQD (Waters). Mass spectrometry analysis was performed on triple quadrupole (Xevo TQD, Waters) in positive ion mode.

At 25 weeks, after 4 hours of fast, mice were anesthetized under isoflurane (5L/min, 2-3%) and blood was collected by cardiac puncture into tubes containing EDTA K2 (Sarsted, Germany). Physiologic sodium chloride solution was injected into systemic circulation. Organs were rapidly excised and snap frozen in liquid nitrogen before being stored at -76°C. Fasting plasma total cholesterol (TC), TG and non-esterified fatty acids (NEFA) were performed using enzymatic assay (Dyasis, Grabels, France). Isolation of lipoproteins was performed by fast protein liquid chromatography (FPLC) (AKTA®, GE Healthcare Europe GmbH, France) using 200 µL of plasma. Plasma apolipoproteins (ApoA-I, ApoB100, ApoC-II and ApoC-III) and gallbladder bile acids (BA) were measured by liquid chromatography-tandem mass spectrometry (LC-MS/MS). Thiobarbituric acid reactive substances (TBARS) content in liver were quantified using the fluorimetric procedure of Yagi (Yagi, K., A simple fluorometric assay for lipoperoxide in blood plasma. Biochem Med, 1976. 15(2): p. 212-6). Plasma choline, betaine, carnitine, TMA and TMAO were analyzed the same way as for 16 weeks old.

### Aortic lesion quantification

The entire aorta linked to the heart was defrosted for microdissection and peripheral fat was completely removed under binocular magnifier. Heart, aortic arch and aorta were separated. Then, for the aortic root lesion quantification, the 2-3 mm long aortic arch connected to a little piece of the left ventricle were frozen in -76°C embedding medium for serial 10-µm-thick cryosectioning. Serial cross-sections of three valves leaflet were sectioned until 850 µm from the heart and were performed at -20°C. Staining and analyses were made on sections corresponding to the beginning of the valves (from 200 µm to 250 µm from the heart), the middle (from 400 µm to 450 µm), the end (from 600 µm to 650 µm) and after (from 800 µm to 850 µm). Neutral lipid quantification, sections were fixed on glass slide with formol 4%, dehydrated with isopropyl alcohol, stained with oil red O and counterstained with hematoxylin. Images of each stained aorta were captured using a digital slide scanner (Nanozoomer Hamamatsu 2.0 HT). Quantification of neutral lipid-stained lesions was performed manually using image analysis software Nanozoomer Digital Pathology View 2. Results were expressed in % as the neutral lipid area quantification (average of 5 sections/distance) reported to the surface of the aortic root.

### Hepatic lipid content

Total hepatic lipids were quantified after a Bligh and Dyer extraction. Briefly, -100 mg of liver was homogenized in 500 µL of NaCl 0.9% and then chloroform/methanol 2/1 (vol/vol) was added. After centrifugation, 200 µL from the organic phase were harvested and transferred into a new tube. Another 500 µL of chloroform/methanol 2/1 (vol/vol) was added in the initial homogenate to realize a second extraction.

Then, organic phase was dried overnight under nitrogen gas and lipids were dissolved in 200 µL of isopropanol/triton 10%. After centrifugation, TG, NEFA and TC were measured in the supernatant with commercial kits (Dyasis, France).

### Short chain fatty acids

Caecal contents were collected for SCFA analysis and mixed with 3 times their weight in water. After centrifugation of thawed samples (10000g for 15 min), SCFA (acetate, propionate, butyrate, and minors; ie, isobutyrate, valerate and isovalerate) concentrations were determined in supernatants diluted with 0.5 mol/L oxalic acid and then analyzed by gas chromatography.

### Real-Time qRT-PCR

Liver RNA extraction was processed using TRizol reagent (Life technologies, France) according to their protocol. After reverse transcription of 1 µg of total RNA realized with SuperScript III Reverse Transcriptase (Life technologies, France) and DNAse treatment (Promega-France, France), samples were analyzed on a Bio-Rad CFX Manager system (Bio-Rad, France).

All primers were ordered from Eurofins: *Peroxisome proliferator-activated receptor alpha (Ppara)* (Forward: 5'- CGTTTGTGGCTGGTCAAGTTCG-3': SEQ ID N°1; Reverse: 5'- AGTGGGGAGAGAGGACAGATGG-3': SEQ ID N°2) and *Tata box binding protein (Tbp)* (Forward: 5'-ACTTCGTGCAAGAAATGCTGAA-3': SEQ ID N°3; Reverse: 5'-GCAGTTGTCCGTGGCTCTCT-3': SEQ ID N°4). Expression data were normalized by the 2(ΔCt) method using *Tbp* as internal control.

### Statistics

Experimental values were presented as the mean ± SEM (standard error to the mean). Statistical analyses were performed using one-way analysis of variance (ANOVA) with Holm-Sidak's multiple comparisons test. Kruskal-Wallis with Dunn's multiple comparisons test was applied to variables not meeting normality and homoscedasticity assumptions. Two-way ANOVA followed by Sidak's multiple comparisons test were applied for statistical comparison of aortic root lesion area over the distances and the follow-up of plasma TG after Triton injection. Correlations were analyzed with Spearman correlation. *p*-values lower than 0.05 were considered significant. All analyses were performed with GraphPad Prism 6 software.

### Results

### Example 1

### Perinatal C-Phycocyanin supplementation prevents offspring atherosclerosis

First, TBARS accumulation was measured in non-gestational ApoE KO mice directly supplemented with Phy to verify antioxidant properties of this concentrate. In order to avoid stress generation in mothers and offspring, blood was not harvested during pregnancy. Phy administration during 3 or 6 weeks, dose dependently decreased plasma TBARS in non-pregnant ApoE KO mice (respectively -41% and -78%) (Figure 1A). No effect was observed on TBARS in liver and caecal content (Fig. 1B and Fig. 1C). Phy administration didn't alter plasma triglyceride and total cholesterol neither (Fig. 1D and Fig. 1E).

Female born to ApoE KO mothers receiving Phy during their gestation (PhyG) or their gestation and lactation (PhyGL) showed a decreased aortic root lesion area compared to control mice at 600 µm from the heart (Control: 0.080 ± 0.010%; PhyG : 0.050 ± 0.010%; PhyGL : 0.051 ± 0.005%) (Fig. 2A, Fig. 2B and Fig. 2D). In male ApoE KO offspring, perinatal administration of Phy statistically reduced aortic root lesion area at 800 µm (Control: 0.054 ± 0.008%; PhyG: 0.010 ± 0.003%; PhyGL: 0.035 ± 0.010%) (Fig. 2A, Fig. 2C and Fig. 2E).

En face analyses of thoracic aortic area in females revealed a significant reduction of lesions in PhyG (-66%, *P*=0.03) and a tendency to lower in PhyGL (-40%, P=0.10) (Fig. 2F). No difference was observed between groups in male offspring (Fig. 2G).

In order to understand underlying mechanisms, we characterized offspring phenotype. Concerning female pups, no difference in weight, in plasma TG and NEFA was measured (Fig. 3A, Fig. 3C and Fig. 3D) but we saw a significant lowering of subcutaneous adipose tissue in PhyGL mice (-28%, P=0.05) (Fig. 3B). Weight and subcutaneous adipose tissue were similar between groups for male (Fig. 3E and Fig. 3F) but plasma TG was enhanced and NEFA was lowered in PhyGL mice (Fig. 3G and Fig. 3H).

In offspring female, although plasma TC was identical between groups (Fig. 4A), plasma lipoprotein quantification after FLPC separation demonstrated that LDL-to-HDL cholesterol ratio was diminished in PhyG (P=0.04) and raised in PhyGL (P=0.02) (Control : 10.54 ± 0.70; PhyG : 8.7 ± 0.62; PhyGL : 12.65 ± 0.40) (Fig. 4B and Fig. 4C). Plasma ApoA-I showed a tendency to rise in PhyG mice (+37%, P=0.08) (Table 1). Liver gene expression of *Peroxisome proliferator-activated receptor alpha (Ppara)* was upregulated in both PhyG and PhyGL groups (respectively +60%, P<0.01 and +37%, P<0.05) (Fig. 4D). Regarding male offspring phenotype, no difference was shown for plasma total cholesterol contrary to LDL-to-HDL cholesterol ratio that tended to decrease in PhyG mice and reduced strongly in PhyGL mice (Control: 10.51 ± 0.64; PhyG : 8.38 ± 0.56; PhyGL : 6.55 ± 0.18) (Figs. 4E to 4G). No difference was observed in plasma apolipoprotein levels (Table 1), neither in liver *Ppara* expression (Fig. 4H).

Hepatic VLDL secretion was estimated after administration of a tyloxapol bolus. Administration of Phy during gestation and lactation alleviated hepatic VLDL secretion rate after 4h of bolus injection in female offspring whereas it increased hepatic VLDL secretion rate in male offspring as soon 3h after tyloxapol injection (Fig. 8A and Fig. 8B). In female, this is accompanied by an accumulation of liver TG in PhyGL and of NEFA in PhyG and PhyGL mice compared to control group (Fig. 8C to 8F) whereas no effect was observed in hepatic lipid composition of offspring male (Fig. 8G to 8J).

Female PhyG or PhyGL showed a slight lowering of plasma TBARS compared to female born to non-supplemented mothers (Fig. 9A). No other effect was seen on TBARS in liver and caecal content in female and male offspring (Fig. 9B to 9F). However, hepatic antioxidative gene expression remained unchanged in female offspring (data not shown) while *Superoxide dismutase1 (Sod1), Catalase* and *Glutathione peroxidase (Gpx)* expressions were all significantly decreased in male PhyG offspring (Fig. 9G to 9I).

To conclude on this example, Phy supplementation during gestation protects against aortic lesion formation.

### Example 2

### C-Phycocyanin modulates offspring bile acids

Gallbladder bile acids (BA) were also investigated because they represent the major cholesterol excretion route from organism. Female BA content was significantly enhanced in PhyG pups compared to control pups (+57%, P<0.01) (Control: 93097 ± 14709 ng/µL; PhyG: 145887 ± 9098 ng/µL; PhyGL: 114419 ± 8624 ng/µL) (Fig. 5A). Hydrophobic BA revealed a tendency to diminish for deoxycholic acid (DCA) in both PhyG and PhyGL mice while tauro-lithocholic acid (LCA) (-36%, *P*=0.09) and glyco-cholic acid (CA) (-32%, *P*<0.05) were lessened in PhyG mice (Fig. 5B to 5D).

No difference in gallbladder BA content was seen in male offspring (Control: 77580 ± 16536 ng/µL; PhyG: 75075 ± 19845 ng/µL; PhyGL: 57542 ± 6890 ng/µL) (Fig. 5E). BA profile in male pups demonstrated a significant lower tauro-chenodeoxycholic acid (CDCA) in PhyG and PhyGL mice (*P*<0.05) and an increase of soluble tauro-ursodeoxycholic acid (UDCA) (+44%, *P*=0.08) and β-muricholic acid (B-MCA) (+116%, *P*<0.05) in PhyGL mice (Fig. 5F to 5H).

These results support the modulation gender specific of gallbladder BA profile by perinatal Phy administration. Perinatal supplemented female pups seem to have different capabilities to metabolize primary to secondary BA.

### Example 3

### Perinatal C-Phycocyanin uptake alters microbial metabolites

As plasma TMAO level has been proposed to be an early cardiovascular marker of ATS, we determined plasma level of TMAO and its precursors in pup's blood at 16 weeks of age. Female PhyGL had significantly less plasma TMAO (Control: 12.6 ± 1.43 µM; PhyG: 11.20 ± 0.95 µM; PhyGL: 8.21 ± 0.97 µM) than control pups (Fig. 6A). TMA, a TMAO's precursor, showed a tendency to be reduced in both PhyG and PhyGL female mice (*P*=0.07) (Fig. 6B). Concerning dietary TMA's precursor, plasma choline was found to be significantly reduced in both PhyG and PhyGL offspring (respectively, -21%, *P*<0.05 and -23%, *P*<0.05) (Fig. 6C).

Male offspring expressed no statistical difference in plasma TMAO (Control: 4.67 ± 0.60 µM; PhyG: 4.00 ± 0.28 µM; PhyGL: 3.68 ± 0.45 µM) and a tendency to decrease in plasma TMA (respectively, -39%, *P*=0.08 and -49%, *P*=0.08) (Figure 6D and E). PhyG male pups showed a reduction in plasma choline (-30%, *P*=0.05) and carnitine (-24%, *P*<0.05) and PhyGL male pups exhibited a lower plasma choline (-25%, *P*=0.07) and betaine (-30%, *P*=0.08) compared to control pups (Fig. 6F to 6H).

We then investigated short chain fatty acids (SCFA) in caecal content knowing they represent the end products of fermentation of dietary fibers by gut microbiota. Direct oral Phy administration during 3 weeks to female ApoE KO mice significantly increased caecal propionate content (Fig. 7A to 7D). In pups of supplemented mothers, there is a significant decrease of total SCFA and a tendency to elevate propionate in female PhyG and PhyGL mice and a lessening of butyrate in PhyG compared to control mice (Fig. 7E to 7H). In offspring male, only a light tendency to alleviate total SCFA in caecal content was reported (Fig. 7I to 7L).

Female aortic root lesion area correlated positively with plasma TMA measured at 16 weeks old (*r*= 0.58, *P*=0.004) and caecal TBARS content (*r*= 0.53, *P*=0.007) and negatively with gallbladder Tauro-DCA (*r*= -0.59, *P*=0.01) and liver *Ppara* expression (*r*= -0.64, *P*=0.001) (Table 2). Male aortic root lesion area correlated positively with plasma LDL-to-HDL ratio (*r*= 0.44, *P*=0.03), plasma TMA measured at 25 weeks old (*r*= 0.44, *P*=0.03), gallbladder Tauro-LCA (*r*= 0.48, *P*=0.04) and liver *Superoxide dismutase 1 (Sod1)* gene expression (*r*= 0.66, P=0.004). Conversely, aortic root lesion area was negatively correlated with liver weight-to-body weight ratio (*r*= -0.42, *P*=0.035), gallbladder Tauro-UDCA (r= -0.49, *P*=0.04) and Glyco-MCA (*r*= -0.51, *P*=0.03).

Collectively, these results stand for a mechanism gender specific involving gut microbiota for the protection of aortic lesion formation induced by perinatal Phy supplementation.

### Discussion

The above examples show protective effect of perinatal C-Phycocyanin administration during gestation or gestation/lactation on atherosclerosis development in female and male offspring of ApoE KO mice. These results show gender specificities regarding potential underlying mechanisms. Female born to supplemented mothers had a greater BA pool, a lower secondary hydrophobic BA level associated with less plasma TMAO and choline levels while male pups expressed a better LDL-to-HDL cholesterol ratio, a more soluble BA pool and a tendency to alleviate TMAO precursors.

In order to avoid stress generation in mothers and offspring, blood was not harvested during pregnancy. Phy administration in non-gestational ApoE KO female mice during 3 or 6 weeks, to mimic the time of gestation or gestation/lactation respectively, strongly and time dependently decreased plasma oxidative stress as reflected by TBARS measurement. Perinatal supplementation with Phy alleviated plasma TBARS measurement in female pups as well. Protective effect of Phy on aortic lesions formation in offspring could be due to a direct effect on plasma lipid, since aortic lesions start to develop in fetus, and/or a better antioxidant protection in mothers.

Perinatal Phy supplementation during lactation didn't bring any apparent additional protective effect to gestational administration regarding aortic lesion development in female and male offspring. This observation suggests that protection effects of Phy on the "programming" of aortic root lesion takes place essentially *in utero.*

Key parameters involved in reverse cholesterol transport, a process preventing cholesterol accumulation in macrophages has been investigated in offspring of supplemented mothers. Phy supplementation diminished plasma LDL-to-HDL cholesterol ratio except in female PhyGL pups. Interestingly, this is associated with an increase of *Ppara* expression only in liver of female offspring mice and a tendency to elevate plasma ApoA-I in PhyG female mice. Furthermore, liver *Ppara* expression negatively correlated with aortic lesion area in female pups. By contrast, if male pups PhyG and PhyGL expressed a decrease of LDL-to-HDL cholesterol ratio, no effect was shown on ApoA-I and hepatic *Ppara* expression meaning probably a different underlying pathway.

Excess peripheral cholesterol is transported in HDL to the liver where it is secreted into the bile. A higher gallbladder BA concentration in female mice born to Phy supplemented mothers suggests a higher cholesterol disposal into feces. If no difference was seen in gallbladder BA pool in male pups, male receiving perinatal Phy expressed higher soluble BA such as Tauro-UDCA and β-MCA. UDCA has been used in the treatment of cholelithiasis because it permits cholesterol solubilization and gallstone dissolution.

Furthermore, the above examples reveal a protection in accumulation of plasma TMAO and its substrates in perinatal Phy supplemented female offspring. A decrease of plasma TMA and its dietary precursors specifically choline, betaine and carnitine is seem. These nutrients can be metabolized by gut microbiota possessing TMA lyase. It has been previously described that diet enriched in choline or TMAO increased aortic root lesion size in the same way (Bennett, B.J., et al., Trimethylamine-N-oxide, a metabolite associated with atherosclerosis, exhibits complex genetic and dietary regulation. Cell Metab, 2013. 17(1): p. 49-60 and Ding, L., et al., Trimethylamine-N-oxide (TMAO)-induced atherosclerosis is associated with bile acid metabolism. Lipids Health Dis, 2018. 17(1): p. 286).

Moreover, it is interesting to note that males have a very less plasma TMAO level than female offspring and inversely a higher plasma TMA level. These results are consistent with the fact that females develop more ATS than male pups. The implication of TMA metabolism products in ATS development is still not fully understood. While some researches associated them with an elevated risk for cardiovascular diseases, other didn't find any correlation with aortic lesion size, macrophage content or plasma cholesterol. Plasma TMAO and its precursors could be an important factor at an early stage of ATS development, at least in ApoE KO mice.

SCFA are mainly produced in the colon by bacterial fermentation of indigestible polysaccharides. They can be absorbed into the bloodstream and exert various metabolic effects. The above results revealed that non pregnant ApoE KO mice supplemented with Phy during 3 weeks have a significant elevation of propionate in their caecal content. This elevation is also found in female offspring. Propionate has been shown to exert anti-inflammatory effects on immune cell functions.

In conclusion, although some differences are noted between males and females descendants, supplementation of phycocyanin to mothers during gestation or during gestation and lactation surprisingly clearly protects descendants against atherosclerosis development, in particular against atheroma formation, or helps in atheroma reduction.

## Claims

1. Composition for use in protecting mammalian subjects against atherosclerosis development, in particular against atheroma formation or in atheroma reduction, by oral administration of said composition comprising an aqueous liquid extract including phycocyanin.

2. Composition according to claim, for use in protecting genetically hypercholestolemic mammalian subjects against atherosclerosis development.

3. Composition according to anyone of claim 1 or 2 in protecting offspring mammalian subjects against atherosclerosis development, by oral administration to mothers of the offspring subjects of said composition comprising an aqueous liquid extract including phycocyanin.

4. Composition according to claim 3, **characterized in that** said composition is administrated at least during a part of the gestation period of the offspring mother.

5. Composition according to anyone of claims 3 or 4, **characterized in that** said composition is administrated at least during part of the gestation period and of the lactation period of the offspring mother.

6. Composition according to anyone of the preceding claims, **characterized in that** said aqueous liquid extract comprises from 0.2 mg to 15 mg, preferably from 0.5 to 12 mg, more preferably from 0.8 to 11 mg of phycocyanin, per mL of extract.

7. Composition according to anyone of the preceding claims, **characterized in that** said composition comprises also polysaccharides from spirulina.

8. Composition according to claim 7, **characterized in that** said aqueous liquid extract comprises from 0.05 mg to 0.25 mg, preferably from 0.10 to 0.20 mg, of polysaccharides from spirulina, per mL of extract.

9. Composition according to anyone of the preceding claims, **characterized in that** said composition is in liquid form and consists essentially of said aqueous liquid extract.

10. Composition according to any one of the preceding claims, **characterized in that** said composition is administrated at a dosage regime of between 1 mg and 100 mg phycocyanin /weight kg/day, preferably between 5 mg and 80 mg phycocyanin /weight kg/day, of the mammalian subject or the offspring mother for animal subjects.

11. Composition according to any one of claims 1 to 9, **characterized in that** said composition is administrated at a dosage regime of between 1 mg and 15 mg phycocyanin /weight kg/day, preferably between 5 mg and 10 mg phycocyanin /weight kg/day, of the human subjects.
